# EUROPÄISCHE PATENTANMELDUNG

(11) **EP 1 228 753 A1**
(43) Veröffentlichungstag der Anmeldung: **07.08.2002**
(21) Anmeldenummer: 02002434.5
(22) Anmeldetag: 01.02.2002
(51) Int. Cl.: A61K 7/50, B65D 81/00

(54) **Kräuterbad**

(30) Priorität: 01.02.2001 DE 20101877 U
(71) Anmelder: Benediktus Kräuterlabor Strathausen GmbH, 84028 Landshut (DE)
(72) Erfinder: Strathausen, Gerold, 84079 Bruckberg (DE)
(74) Vertreter: Pausch, Thomas, Dipl.-Phys.

(57) **Zusammenfassung**

Die Erfindung bezieht sich auf ein Kräuterbad, enthaltend Heil- oder Naturkräuter (2) und/oder aromatisierende Extrakte (3) in getrockneter bzw. fester Form als Wirkstoffe, und einen die Wirkstoffe umhüllenden Beutel (4) aus wasserdurchlässigem und gegenüber heißem Wasser beständigen, reißfesten Material. Der die Wirkstoffe (2, 3) umhüllende Beutel (4) besteht aus einem Viskosevlies, insbesondere reinem Viskosevlies, oder aus einem Zellulose-, Papier- oder Stärkematerial. Die Wirkstoffe (2, 3) umfassen nur natürliche Kräuter und auf die Kräuter aufgebrachte oder mit den Kräutern angereicherte, insbesondere diesen entsprechende hochwertige natürliche oder reine ätherische Öle und/oder Extrakte. Neben den Wirkstoffen ist ein Farbstoff, insbesondere ein natürlicher Farbstoff sowie ein weiterer Duftstoff, insbesondere natürliche Duftöle oder eine Duftkomposition enthalten. Das Kräuterbad ist einzeln in einer luftdichten Folie (6) aus durchsichtigem oder wenigstens durchscheinenden Material verpackt ist.

## Beschreibung

Die Erfindung bezieht sich auf ein Kräuterbad enthaltend Heil- oder Naturkräuter und /oder aromatisierende Öle oder Extrakte als Wirkstoffe zur Verwendung in einem Badewasser.

Der Brauch, das Badewasser mit Kräuterölen und Extrakten zu aromatisieren, ist so alt wie die Badekultur selbst. Kräuterbäder sind Balsam für Körper, Seele und Geist. Bereits die schönen Frauen der Antike wussten die Vorzüge eines duftenden Melissenbades zu schätzen. Und der berühmte Kräuter-Pfarrer Kneipp hat die Wirkungen von pflanzlichen Zusätzen im Badewasser wiederentdeckt. Heutzutage kann man überall Kräuterund Schaumbäder kaufen und diese zu Hause anwenden. Aber chemische Zusätze wie Duft-, Farb- und Konservierungsstoffe belasten die Haut des Badenden, so dass der positive Effekt der ätherischen Wirkstoffe und Öle stark beeinträchtigt wird.

Außerdem sind Kräuterbäder meist nur in flüssiger oder pulveriger Form erhältlich. Wegen der damit verbundenen Gefahr des Auslaufens von Flüssigkeit oder Pulver bei Beschädigung der Verpackung sind solche Kräuterbäder insbesondere zur Mitnahme auf Reisen weniger geeignet. Schließlich ist die Dosierung solcher flüssiger oder pulveriger Kräuterbäder zumindest erläuterungsbedürftig und erfordert weitere Hilfsmittel wie Messbecher oder dergleichen, auch wenn diese oftmals beispielsweise in den Verpackungsverschlüssen integriert sind.

Der vorliegenden Erfindung liegt die Aufgabe zugrunde, ein Kräuterbad der gattungsgemäßen Art zur Verfügung zu stellen, welches neben einer einfacheren Handhabung bzw. Dosierung auch zur Mitnahme für Unterwegs besser geeignet ist.

Die Lösung der Aufgabe erfolgt durch ein Kräuterbad nach den nebengeordneten Ansprüchen 1 und 2.

Nach dem erfindungsgemäßen Gegenstand nach Anspruch 1 liegen die Wirkstoffe des Kräuterbades, nämlich die Heil- oder Naturkräuter und/oder die aromatisierenden Extrakte, in getrockneter bzw. fester Form vor, und des weiteren ist ein die Wirkstoffe umhüllender Beutel vorgesehen, der aus wasserdurchlässigem und gegenüber heißem Wasser beständigen, reißfesten Material besteht. Der Erfindung nach Anspruch 1 liegt die Grundidee zugrunde, ein Kräuterbad gleichsam in der Form eines Teebeutels für die Badewanne vorzusehen. Das erfindungsgemäße Kräuterbad in Form eines "Badebeutels" gewährleistet ein Kräuterbaden in einer duftenden Badewanne ohne der unangenehmen Eigenschaften bislang bekannter Kräuterbäder. Die Menge der Wirkstoffe eines einzelnen Kräuterbades nach der Erfindung ist entsprechend einem durchschnittlichen Vollbad dosiert und erübrigt dadurch jegliches lästiges und ungenaues Dosieren wie bei den vorbekannten Kräuter- oder Schaumbädern. Eine (oder auch mehrere) einzelne Verpackung des erfindungsgemäßen Kräuterbades lässt sich problemlos für unterwegs mitnehmen und findet etwa in einer Damenhandtasche Platz.

Nach dem erfindungsgemäßen Gegenstand nach dem nebengeordneten Anspruch 2 liegen die Wirkstoffe des Kräuterbades, nämlich die Heil- oder Naturkräuter und/oder die aromatisierenden Extrakte, gleichfalls in getrockneter bzw. fester Form vor, und zwar als wasserlösliche Wirkstoffe, wobei der die Wirkstoffe umhüllende Beutel aus einem in heißem Wasser löslichen bzw. auflösbaren Material besteht. Auch der Erfindung nach Anspruch 2 liegt die Grundidee zugrunde, ein Kräuterbad gleichsam in der Form eines Teebeutels für die Badewanne vorzusehen, nur ist hier das Kräuterbad mitsamt seiner Umhüllung aus einem Material bzw. einer Substanz, welche in heißem Wasser von beispielsweise 36 bis 38 °C jedenfalls nach einer gewissen Zeit von einigen Minuten vollständig aufgelöst ist. Auch das erfindungsgemäße Kräuterbad nach Anspruch 2 in Form eines "Badebeutels" gewährleistet ein Kräuterbaden in einer duftenden Badewanne ohne der unangenehmen Eigenschaften bislang bekannter Kräuterbäder. Die Menge der Wirkstoffe eines einzelnen Kräuterbades nach der Erfindung ist entsprechend einem durchschnittlichen Vollbad dosiert und erübrigt dadurch jegliches lästiges und ungenaues Dosieren wie bei den vorbekannten Kräuter- oder Schaumbädern. Eine (oder auch mehrere) einzelne Verpackung des erfindungsgemäßen Kräuterbades lässt sich problemlos für unterwegs mitnehmen und findet etwa in einer Damenhandtasche Platz.

Zur Anwendung wird das erfindungsgemäße Kräuterbad mit dem die Wirkstoffe umhüllenden Beutel einfach in die Badewanne gelegt, heißes Wasser einlaufen gelassen und die Wassertemperatur nach der gewünschten Temperatur des Badenden reguliert. Durch die Benetzung oder Auflösung der Wirkstoffe mit dem heißen Badewasser werden die Wirkstoffe und herrlich duftenden Öle langsam und in ihrer vollen Wirkung freigesetzt und garantieren somit ein hervorragendes und vor allem auch gesundes Badevergnügen.

Ein mit Wasser getränktes Kräuterbad kann auch ausgezeichnet zur Massage oder auch als Kompresse verwendet werden. Hierbei ist aufgrund der hochwertigen Struktur des Beutels keinerlei Gefahr eines Zerreißens gegeben.

Weiterhin kann das erfindungsgemäße Kräuterbad mit dem Beutel auch in der Sauna verwendet werden. Für die Anwendung der heilenden Wirkungen der ätherischen Öle ist der Beutel mit den darin enthaltenen Wirkstoffen einfach in die Sauna zu legen, wodurch die Wirkstoffe und ätherischen Öle aufgrund der in der Sauna herrschenden Temperatur und Feuchtigkeit freigesetzt werden.

Weitere Anwendungen des erfindungsgemäßen Kräuterbades etwa in der Aromatherapie sind durchaus beabsichtigt.

Die im erfindungsgemäßen Kräuterbad enthaltenen bevorzugten duftenden Heil- oder Naturkräuter entsprechen dem hohen Standart des deutschen Arzneibuches und sind demzufolge frei von Rückständen oder zumindest rückstandskontrolliert. Hochwertige reine ätherische Öle und Extrakte sind mit aufwändigen Verfahren auf die Heil- oder Naturkräuter aufgebracht. Die auf den Heil- oder Naturkräutern aufgebrachten Duftkomponenten können hierbei aus verschiedenen ätherischen Ölen bestehen. Beispielsweise gibt es bei Kamille kein reines Kamillenöl, welches bezahlbar wäre. Vielmehr werden hervorragende Mischungskomponenten verwendet, die stabil und gut duftend sind. Dennoch handelt es sich hierbei um reine ätherische Öle im Sinne der Erfindung. Auf diese Weise kann von Baden in "reiner Natur" gesprochen werden, so dass die Natürlichkeit der Düfte hervorgehoben wird.

Die Wirkstoffe des erfindungsgemäßen Kräuterbades umfassen somit natürliche Kräuter und auf die Kräuter aufgebrachte oder mit den Kräutern angereicherte, insbesondere diesen entsprechende hochwertige natürliche oder reine ätherische Öle und/oder Extrakte. Beispielsweise enthält das Kräuterbad hochwertige natürliche Kamillenkräuter, die zusätzlich mit deren wertvollen natürlichen ätherischen Ölen angereichert sind, die dem Bad eine wohltuende und pflegende Komponente verleihen.

Nach einer bevorzugten Weiterbildung der Erfindung ist neben den Wirkstoffen ein Farbstoff, insbesondere ein natürlicher Farbstoff enthalten. Die Farbstoffe sind in einem komplexen Verfahren entweder auf den Wirkstoffen aufgebracht oder mit den Wirkstoffen angereichert, oder einfach mit den Wirkstoffen gemischt. Die im Kräuterbad enthaltenen Farbstoffe bewirken die gewünschte Einfärbung des Badewassers passend zu der Art der enthaltenen Wirkstoffe und sorgen auf diese Weise für den entsprechenden optischen Effekt für den Benutzer.

Bei einer besonders bevorzugten Ausgestaltung der Erfindung beträgt der Kräuteranteil in dem Kräuterbad etwa 5 bis 75 g, insbesondere 10 bis 50 g, und insbesondere 15 bis 20 g.

Bei einer weiterhin bevorzugten Ausgestaltung der Erfindung beträgt der Anteil der ätherischen Öle und/oder Extrakte am Gesamtgewicht einer einzelnen Packung eines Kräuterbades etwa 5 bis 25, und insbesondere etwa 15 bis 20 Gewichts-% der Gesamtmasse eines Kräuterbades.

Der die Wirkstoffe umhüllende Beutel des Kräuterbades besteht vorzugsweise aus einem Viskosevlies, insbesondere reinem Viskosevlies, oder aus einem Zellulose-, Papier- oder Stärkematerial als natürlichem Stoff, vorzugsweise ohne künstliche chemische Zusätze.

Der die Wirkstoffe umhüllende Beutel weist eine Breite von etwa 8 bis 20, insbesondere 10 bis 15 cm, und insbesondere etwa 12 bis cm, und eine Länge von 12 bis 16 cm, insbesondere etwa 14 cm aufweist.

Bei einem weiteren Ausführungsbeispiel kann vorgesehen sein, dass der Beutel mehrteilig ausgebildet ist oder aus mehreren, untereinander getrennten Kammern besteht. Hierbei kann eine Kammer die Heil- oder Naturkräuter und/oder aromatisierende Extrakte in getrockneter bzw. fester Form, und die andere(n) Kammer(n) die ätherischen Öle und/oder die Farbstoffe und/oder Duftstoffe bzw. deren Komponenten oder Zusammensetzungen enthalten.

Viele medizinisch wirksame Inhaltsstoffe werden während des Badens direkt über die Haut aufgenommen, da die Poren weit gestellt sind. Beim Einatmen gelangen die ätherischen Öle, die sich wunderbar mit dem Wasserdampf in der Raumluft verteilen, über die Bronchien in die Lunge und so in den Blutkreislauf. Sie strömen zu den verschiedenen Organen und entfalten dort ihre heilkräftige Wirkung. Gegenüber den bisherigen Bädern wird durch das erfindungsgemäße Kräuterbad gerade auch die Aufnahme der ätherischen Öle über die Raumluft und damit die Heilwirkung verbessert.

Von weiterem Vorteil kann vorgesehen sein, dass das Kräuterbad einzeln in einer luftdichten Folie aus durchsichtigem oder wenigstens durchscheinenden Material verpackt ist. Jedes einzelne Kräuterbad nach der Erfindung ist von Vorteil beispielsweise in einer beschichteten Polypropylenfolie einzelverpackt. Gegenüber Folien aus anderen Materialien, etwa Papier, Aluminium, oder Polyethylen besteht neben dem Preisvorteil vor allem auch eine wesentlich verbesserte Aromadichtigkeit bei PP-Folien. Außerdem ist die Entsorgungsproblematik geringer; der Wirkstoff Polypropylen kann problemlos im dualen System oder jedem anderen Recyclingsystem entsorgt werden, und ist als Einkomponentenverpackung im Hinblick auf eine Packmittelabgabe zu bevorzugen.

Aufgrund der Durchsichtigkeit der Verpackung wird dem Verbraucher ein direkter Blick auf das Produkt gestattet, so wie er es auch von Produkten aus der Lebensmittelbranche kennt.

Weitere Vorteile der Erfindung ergeben sich aus den Unteransprüchen.

Weitere Zweckmäßigkeiten, Vorteile und Merkmale der Erfindung ergeben sich aus der Beschreibung eines bevorzugten Ausführungsbeispieles anhand der Zeichnung. Es zeigt:

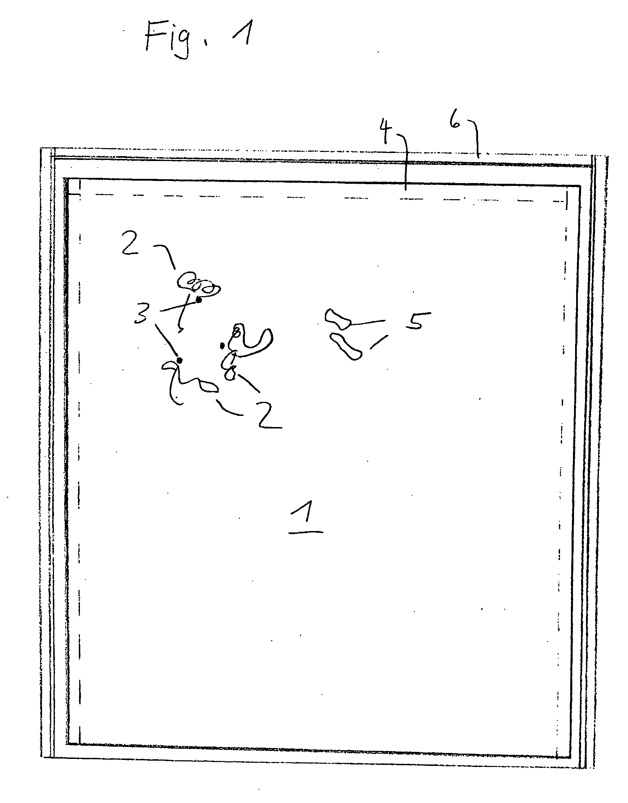

- Figur 1: ein bevorzugtes Ausführungsbeispiel des erfindungsgemäßen Kräuterbades.

Das in Figur 1 schematisch dargestellte Ausführungsbeispiel eines erfindungsgemäßen Kräuterbades 1 enthält Heil- oder Naturkräuter 2 und/oder aromatisierende Extrakte 3 in getrockneter bzw. fester Form als Wirkstoffe, sowie einen die Wirkstoffe umhüllenden Beutel 4 aus wasserdurchlässigem und gegenüber heißem Wasser beständigen, reißfesten Material. Der die Wirkstoffe umhüllende Beutel 4 besteht aus reinem Viskosevlies. Neben den Wirkstoffen 2, 3 ist ein Farbstoff 5, insbesondere ein natürlicher Farbstoff im Kräuterbad 1 enthalten. Das Kräuterbad bestehend aus Beutel 4 und darin enthaltenden Wirkstoffen 2 und 3 ist in einer luftdichten Folie 6 aus durchsichtigem oder wenigstens durchsichtigen Material einzeln verpackt, wobei die Einzelverpackung vorzugsweise aus einer transparenten beschichteten Polyethylenfolie besteht.

## Patentansprüche

1. Kräuterbad, enthaltend Heil- oder Naturkräuter (2) und/oder aromatisierende Extrakte (3) in getrockneter bzw. fester Form als Wirkstoffe, und einen die Wirkstoffe umhüllenden Beutel (4) aus wasserdurchlässigem und gegenüber heißem Wasser beständigen, reißfesten Material.

2. Kräuterbad, enthaltend Heil- oder Naturkräuter (2) und/oder aromatisierende Extrakte (3) in getrockneter bzw. fester Form als wasserlösliche Wirkstoffe, und einen die Wirkstoffe umhüllenden Beutel (4) aus einem in heißem Wasser löslichen bzw. auflösbaren Material.

3. Kräuterbad nach Anspruch 1 oder 2,
**dadurch gekennzeichnet,**
**dass** der die Wirkstoffe (2, 3) umhüllende Beutel (4) aus einem Viskosevlies, insbesondere reinem Viskosevlies, oder aus einem Zellulose-, Papier- oder Stärkematerial besteht.

4. Kräuterbad nach Anspruch 1, 2 oder 3,
**dadurch gekennzeichnet,**
**dass** die Wirkstoffe (2, 3) nur natürliche Kräuter und auf die Kräuter aufgebrachte oder mit den Kräutern angereicherte, insbesondere diesen entsprechende hochwertige natürliche oder reine ätherische Öle und/oder Extrakte umfassen.

5. Kräuterbad nach einem der vorhergehenden Ansprüche,
**dadurch gekennzeichnet,**
**dass** neben den Wirkstoffen ein Farbstoff, insbesondere ein natürlicher Farbstoff enthalten ist.

6. Kräuterbad nach einem der vorhergehenden Ansprüche,
**dadurch gekennzeichnet,**
**dass** neben den Wirkstoffen ein weiterer Duftstoff, insbesondere natürliche Duftöle oder eine Duftkomposition enthalten ist.

7. Kräuterbad nach einem der vorhergehenden Ansprüche,
**dadurch gekennzeichnet,**
**dass** es einen Kräuteranteil von etwa 5 bis 75 g, insbesondere 10 bis 50 g, und insbesondere 15 bis 20 g enthält.

8. Kräuterbad nach Anspruch 3,
**dadurch gekennzeichnet,**
**dass** der Anteil der ätherischen Öle und/oder Extrakte etwa 5 bis 25, und insbesondere etwa 15 bis 20 Gewichts-% der Gesamtmasse eines Kräuterbades (1) umfasst.

9. Kräuterbad nach einem der Ansprüche 1 bis 5,
**dadurch gekennzeichnet,**
**dass** der die Wirkstoffe umhüllende Beutel (4) eine Breite von etwa 8 bis 20, insbesondere 10 bis 15 cm, und insbesondere etwa 12 bis cm, und eine Länge von 12 bis 16 cm, insbesondere etwa 14 cm aufweist.

10. Kräuterbad nach einem der vorhergehenden Ansprüche,
**dadurch gekennzeichnet,**
**dass** es einzeln in einer luftdichten Folie (6) aus durchsichtigem oder wenigstens durchscheinenden Material verpackt ist.

11. Kräuterbad nach einem der vorhergehenden Ansprüche,
**dadurch gekennzeichnet,**
**dass** der Beutel (4) mehrteilig ausgebildet ist oder aus mehreren, untereinander getrennten Kammern besteht.
